# EUROPÄISCHE PATENTSCHRIFT

(11) **EP 3 081 180 B1**
(45) Veröffentlichungstag und Bekanntmachung des Hinweises auf die Patenterteilung: **21.10.2020**
(21) Anmeldenummer: 16173279.7
(22) Anmeldetag: 24.06.2011
(51) Int. Cl.: A61B 17/80, A61B 17/86, A61B 17/00

(54) **FIXATIONSSYSTEM FÜR KNOCHEN**
FIXATION SYSTEM FOR BONES
SYSTEME DE FIXATION POUR OS

(30) Priorität: 30.06.2010 DE 102010025702
(43) Veröffentlichungstag der Anmeldung: 19.10.2016
(62) Teilanmeldung aus: 11730209.1
(73) Patentinhaber: aap Implantate AG, 12099 Berlin (DE)
(72) Erfinder: Paulin, Thomas, 14522 Wilhelmshorst-Michendorf (DE); Bartsch, Thomas, 10367 Berlin (DE); Fischer, Hans-Joachim, 12277 Berlin (DE); Alemu, Bruke Seyoum, 14195 Berlin (DE)
(74) Vertreter: Friderichs, Gunther

(56) Entgegenhaltungen:
- EP-A1- 1 649 819
- EP-A1- 1 859 752
- EP-A1- 2 016 918
- EP-A2- 1 949 866
- WO-A1-97/09000
- WO-A2-2004/084701
- WO-A2-2007/014192
- DE-A1- 19 858 889
- FR-A1- 2 880 929

## Beschreibung

### Gebiet der Erfindung

Die Erfindung bezieht sich auf ein Fixationssystem für Knochen mit Knochenschrauben und einer Knochenplatte, die mehrere in Richtung der Plattenlängsachse angeordnete Löcher für die Aufnahme von Knochenschrauben aufweist.

### Hintergrund der Erfindung

Bei einer bekannten Knochenplatte (EP 0760632 B1) sind die Löcher zur Oberseite der Knochenplatte hin rundum sphärisch ausgebildet, um eine Knochenschraube mit kugeliger Unterseite des Kopfes in unterschiedlichen Winkelstellungen abstützen zu können. An der Plattenunterseite benachbart zum Knochen weisen die Löcher einen Bereich kleineren Durchmessers mit partiellem Gewinde auf, um auch eine Knochenschraube mit zylindrischem Gewindekopf aufnehmen zu können, die senkrecht zur Plattenebene eingesenkt werden soll.

Bei einer weiteren Knochenplatte (EP 1158915 B1 und EP 1158916 B1) sind Langlöcher mit einem Innengewinde, das sich an einem Ende des Langlochs von der Oberseite bis zur Unterseite der Knochenplatte erstreckt und einen Umfangs- oder Zentriwinkel im Bereich von 190-280°aufweist, vorhanden. Das Innengewinde nimmt die gesamte Tiefe des Langloches ein, verjüngt sich gegen die Unterseite der Knochenplatte hin konisch und weist einen Konuswinkel im Bereich von 5 bis 20°auf.

Bei einer weiteren bekannten Knochenplatte (EP 1255498 B1) sind Langlöcher in der Knochenplatte vorgesehen, die oval, elipsenförmig oder auch rechteckig ausgebildet sein können oder eine Kombination solcher Formen beinhalten; lediglich kreisrunde Löcher sind von dieser Definition des Langlochs explizit ausgeschlossen worden. Das Langloch ist mit einem kreisförmigen Loch kombiniert und dieses ist mit einer dreidimensionalen Strukturierung versehen, welche in Form eines Innengewindes oder einer peripheren Lamelle oder Lippe vorliegt. Dargestellt ist ein konisches Innengewinde, das sich von der Oberseite bis zur Unterseite der Knochenplatte erstreckt und einen Umfangs- oder Zentriwinkel im Bereich von 190 bis 280° aufweist.

Aus DE 19858889 A1 ist ein Fixationssystem für Knochen mit einer Knochenplatte bekannt, welche Langlöcher als Durchgangslöcher besitzt, die nahe ihrer dem Knochen benachbarten Unterseite Vorsprünge aufweist, welche sich im unteren Teil des Langloches parallel zur Plattenebene erstrecken. Zur Oberseite der Knochenplatte hin gibt es Sitzflächen für Kugelköpfe der Knochenschrauben. Die Sitzflächen sind als nach innen geneigte Konusflächen oder Kugelflächen ausgebildet. Um mit den Vorsprüngen in der Knochenplatte zusammen zu arbeiten, weist die Knochenschraube unterhalb des Kugelkopfes ein kurzes Stück Gewinde auf, das in der Lage ist, die nachgiebigen Vorsprünge in dem Langloch zu deformieren und sich anzuverwandeln. Die Sitzflächen der Knochenplatte und der Knochenschraube machen es möglich, die Knochenschraube in verschiedenen Winkelstellungen bezüglich der Achse der Durchgangslöcher festzulegen.

Das Dokument EP 1 859 752 A1 zeigt ein Fixationssystem mit einer Knochenplatte und einer Knochenschraube, bei welchem eine winkelstabile Verbindung bei gleichzeitiger Kompression der Knochen ermöglicht ist.

### Allgemeine Erfindungsbeschreibung

Der Erfindung liegt die Aufgabe zugrunde, ein Fixationssystem mit Knochenschrauben und einer Knochenplatte zu schaffen, bei dem verschiedenartige Knochenschrauben - solche mit Konussitzflächen und solche mit Kugelsitzflächen - angewendet werden können, um den unterschiedlichen Anforderungen beim Fixieren eines gebrochenen oder geschädigten Knochens zu genügen. Insbesondere sollen Knochenfragmente beim Fixieren relativ zueinander verschoben werden können.

Das neue Fixationssystem ist aus zwei Arten Knochenschrauben und einer Knochenplatte aufgebaut, wobei mit der ersten Art Knochenschrauben nur Verschraubung senkrecht zur Plattenform der Knochenplatte möglich ist, die zu diesem Zweck eine radiale Rippe und eine konische Sitzfläche aufweist, während mit der zweiten Art Knochenschraube im Schrägwinkel zur Plattenebene gearbeitet werden kann, wobei kein Eingriff der Knochenschraube in die radiale Rippe erfolgt. Die zweite Art der Knochenschraube weist teilsphärische Flächen an der Kopfunterseite auf, jedoch kein Gewinde zum Eingriff in die Knochenplatte.

Im Einzelnen weist die Knochenplatte einen vorzugsweise länglichen Plattenkörper aus einem ersten, gewebeverträglichen, steifen Material auf, der eine Oberseite und Unterseite und eine Längsachse bestimmt. Quer zur Plattenebene sind Lochausbildungen vorgesehen, die aus einem ersten, größeren Rundloch und einem zweiten, kleineren Rundloch bestehen, wobei sich die Rundlöcher unter Bildung von Kanten schneiden, zwischen denen ein Durchlass für den Schraubenschaft einer schräg angesetzten Knochenschraube gebildet wird. Um beide Rundlöcher herum ist eine radiale Rippe vorgesehen, die von der Lochwandung ausgeht und sich in einer Ebene nahe der Schraubeneinführungsseite auf die Rundlochmitte hin erstreckt. Die erste Art der Knochenschraube ist mit Gewinde am Schraubkopf versehen und kann sich gegebenenfalls an der Rippe des kleineren Rundloches abstützen und sich mit der Rippe des größeren Rundloches verschrauben, wodurch Deformation und gegenseitige Formanpassung erfolgt.

Im Sinne der Erfindung behält diese Deformation auch nach dem Trennen der Knochenschraube von der Knochenplatte einen bleibenden Anteil, dies bedeutet, dass diese Deformation über den rein elastischen Anteil hinaus auch plastische Deformationen umfasst.

Dieser plastisch deformierte, bleibende Anteil der Deformation kann je nach Material und Ausgestaltung der konstruktiven Abmessungen allein in der Knochenschraube vorliegen, kann allein in der Knochenplatte vorliegen oder kann auch in beiden, sowohl der Knochenschraube als auch der Knochenplatte vorliegen.

Typischerweise liegt der plastische, verbleibende Anteil in jeweils einer Art Furche oder Rille vor, welche mindestens eine Tiefe von 10 µm, bevorzugt von mehr als 100 µm und besonders bevorzugt von mehr als 200 µm aufweist.

Gemäß bevorzugter Ausführungsform weist das erste, größere Rundloch drei Abschnitte auf, nämlich einen oberen, rundkehlförmigen Abschnitt oberhalb der umlaufenden Rippe, einen mittleren, rundkehlförmigen Abschnitt unterhalb der umlaufenden Rippe sowie einen unteren, kegelstumpfförmigen Abschnitt, der sich zur Unterseite des Plattenkörpers hin verjüngt und dessen größter Durchmesser kleiner als der Durchmesser des oberen oder mittleren Abschnitts ist. Das kleinere Rundloch umfasst einen oberen Abschnitt mit einem geneigten Übergangsbereich zur Plattenoberseite hin, ferner einen mittleren Abschnitt unterhalb der Ebene der umlaufenden Rippe mit geneigt-abgerundeter Fläche sowie einen unteren, zylindrischen oder konischen Abschnitt mit einem Durchmesser kleiner als der Durchmesser des oberen oder mittleren Abschnitts.

Bei allen Ausführungsformen lassen sich Knochenschrauben der ersten Art mit einem Kopf verwenden, der in seinem oberen Bereich mit Schraubgewinde und in seinem unteren Bereich mit einer konischen Sitzfläche versehen ist, und zwar in der Weise, dass beim exzentrischen Einschrauben senkrecht zur Plattenebene in einen Knochen eine Verschiebung des Kopfes relativ zu der Lochausbildung stattfindet, dergestalt, dass Knochenbruchstücke beim Fixieren aneinander angenähert werden können. Die neue Knochenplatte ermöglicht aber auch die Anwendung von Knochenschrauben der zweiten Art mit sphärischen Sitzflächen an der Unterseite des Schraubkopfes. Solche Kugelkopfschrauben können im Winkel zu der Knochenplatte eingeschraubt werden, wobei der Schraubenschaft im Knochen verankert wird und die Fixierung durch Anlage der sphärischen Sitzfläche des Kopfes an passender Sitzfläche der Knochenplatte erfolgt.

Weitere Einzelheiten der Erfindung ergeben sich aus der Beschreibung der dargestellten Ausführungsbeispiele und den angefügten Ansprüchen.

### Die Zeichnungen zeigen:

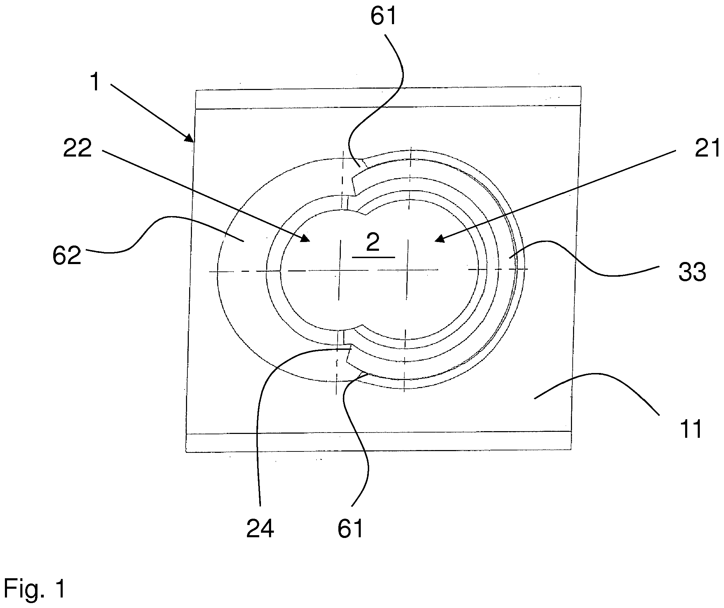

Fig. 1 eine Draufsicht auf eine Lochausbildung in einer Knochenplatte,

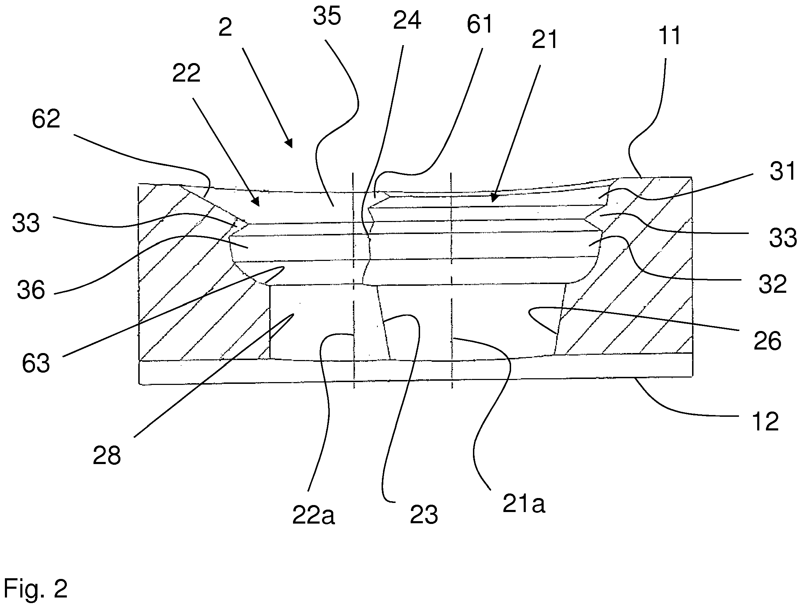

Fig. 2 eine Schnittansicht der Lochausbildung,
Fig. 3 eine perspektivische Darstellung der Lochausbildung,
Fig. 4 eine Knochenschraube im Eingriff mit einer Lochausbildung,
Fig. 5 eine zweite Ausführungsform einer Knochenplatte in perspektivischer Darstellung,
Fig. 6 eine Schnittansicht der Lochausbildung,
Fig. 7 eine Seitenansicht der Lochausbildung, und
Fig. 8 eine Knochenschraube im Eingriff mit einer Lochausbildung, zusammen mit einer Ausschnittsvergrößerung.

### Detaillierte Beschreibung bevorzugter Ausführungsformen

Die Figuren zeigen das Stück einer Knochenplatte, die aus einem länglichen Plattenkörper 1 aus einem ersten, gewebeverträglichen, steifen Material besteht und in den eine Reihe von Lochausbildungen angebracht sind, von denen eine Lochausbildung 2 dargestellt ist. Der Plattenkörper 1 kann außer länglich auch oval, rund oder mehreckig ausgebildet oder von seiner Form her der jeweiligen Anwendung angepasst sein.

Als gewebeverträgliches Material werden beispielsweise Metalle und deren Legierungen verstanden, wie diese üblicherweise zur Herstellung von Implantaten verwendet werden. Bevorzugte Metalle umfassen Titan in jeder Form, vorzugsweise auch dessen Legierungen TiAl6V4 und TiCp..

Auch Stähle, wie beispielsweise Implantatestahl, beispielsweise die Legierung 1.4441 sind vorteilhaft verwendbar.

Eine weitere Materialklasse umfasst hierfür auch resorbierbare Materialien, wie beispielsweise Magnesium oder PLA. PLA ist ein biokompatibler und resorbierbarer Kunststoff aus chemisch aneinander gebundenen Milchsäuremolekülen, der wie auch andere resorbierbare Kunststoffe verwendet werden kann.

Als steif wird eine Knochenplatte dann angesehen, wenn diese konstruktiv die für ihre vorgesehene Verwendung und Einsatzzweck nötige Steifigkeit zur Verfügung stellt. Dies kann je nach verwendetem gewebeverträglichen Material durch die Dicke und Breite der Platte sichergestellt werden und liegt üblicherweise im Bereich fachmännischen Handelns.

So sind beispielsweise Knochenplatten für kleine und weniger belastete Körperpartien, wie beispielsweise Hand- und Fußknochen dünner und häufig weniger breit als Knochenplatten der größeren und stärker belasteten Partien, wie beispielsweise bei Teilen des Unter- und des Oberschenkels.

Die Knochenplatte weist eine Oberseite 11 und eine Unterseite 12 auf, die gewöhnlich parallel zur Plattenebene verlaufen, wobei die Unterseite 12 dem zu fixierenden Knochen benachbart ist. Die Lochausbildungen 2 sind entlang der Längsachse des länglichen Plattenkörpers 1 aufgereiht und bestehen aus zwei stufig ausgebildeten Rundlöchern, 21 und 22, die sich quer zur Plattenebene erstrecken und deren Achsen 21a, 22a die Längsachse der Knochenplatte schneiden. Der kleinste Durchmesser des ersten Rundlochs 21 ist größer als der kleinste Durchmesser des zweiten Rundlochs 22 und der Abstand der beiden Achsen 21a und 22a voneinander ist kleiner als der kleinste Durchmesser des ersten Rundlochs 21.

Durch die Überschneidung der Löcher 21, 22 ergeben sich Kanten 23, 24, welche die Bereiche der Rundlöcher gegeneinander abgrenzen und einen Durchlass für den relativ dünnen Schraubschaft einer Knochenschraube freilassen, wenn bei der Schrägstellung einer Knochenschraube diese von einem Rundloch in das andere Rundloch hineinreicht. Die Umfangswinkel der Wandlaibungen der Rundlöcher 21, 22 betragen ca. 250° des größeren Loches und ca. 220° des kleineren Loches. Variationen um 10° kleiner und 20° größer sind möglich.

Infolge der Stufigkeit der Rundlöcher 21, 22 kann man eine obere Region 25 und eine untere Region 26 des Rundloches 21 und eine obere Region 27 sowie eine untere Region 28 des Rundlochs 22 unterscheiden.

Die oberen Regionen 25 und 27 sind schüsselförmig ausladend gestaltet, während die unteren Regionen 26 und 28 Mantelflächen mit geraden Mantellinien bilden. Die oberen Regionen 25 und 27 weisen größere Durchmesser gegenüber den unteren Regionen 26 und 28 auf. Die untere Region 26 bildet einen kegelstumpfförmigen Abschnitt, der sich zur Unterseite des Plattenkörpers 1 hin verjüngt. Die untere Region 28 ist zylindrisch ausgebildet, sie kann aber auch kegelstumpfförmig ausgebildet sein.

Während die beiden oberen Regionen 25 und 27 der beiden Rundlöcher 21, 22 insgesamt schüsselförmig gestaltet sind, zieht sich, ausgehend von den Lochwandungen, eine radiale Rippe 33 in einer Ebene um die Lochausbildung 2 herum. Die Rippe 33 ist als umlaufender Steg ausgebildet und weist einen keilförmigen Querschnitt auf, der sich zur Mitte des jeweiligen Rundloches hin verjüngt. In der Draufsicht erscheint die Rippe 33 einer Acht ähnlich zu sein.

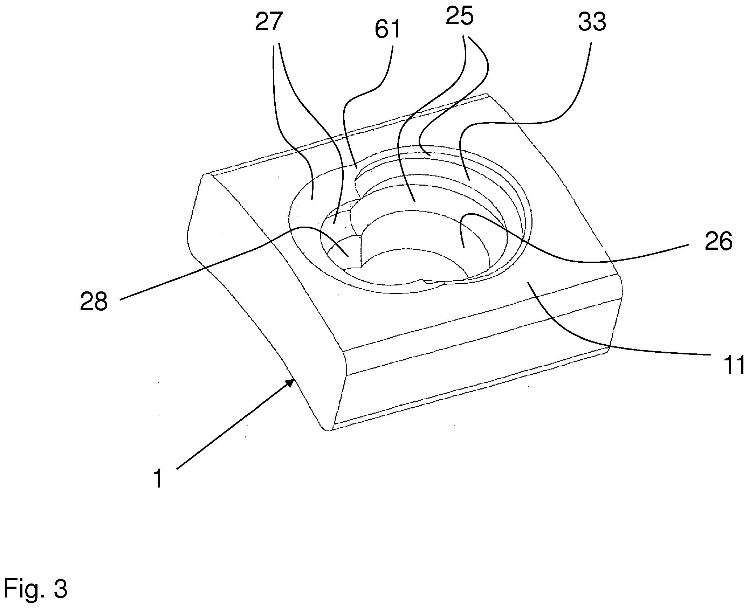

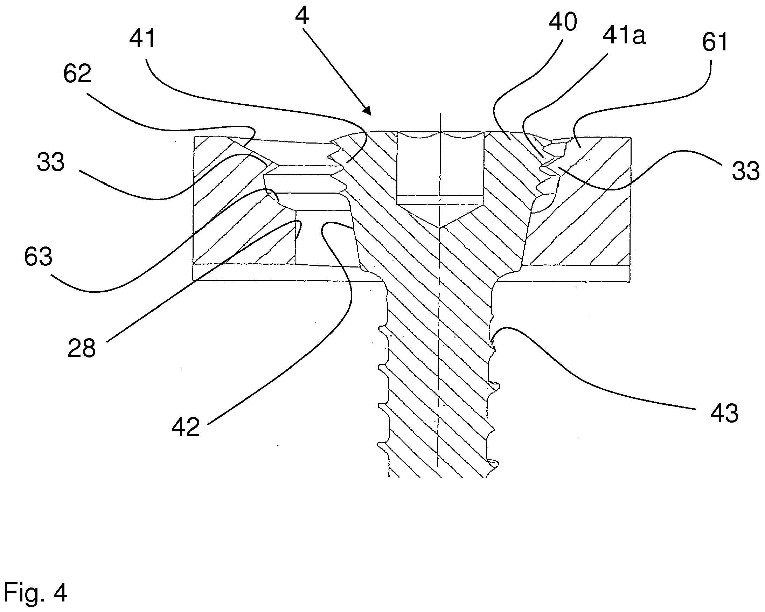

Während die Rippe 33 gleichmäßig herumlaufen kann, wird für den am größeren Rundloch angebrachten Rippenteil ein Grat 61 parallel zur Ebene der Rippe 33 am Außenrand des größeren Rundlochs 21 angeordnet, wodurch sich eine Kante 24 zum zweiten Rundloch 22 hin bildet. Ausgehend von der Längsachse des Plattenkörpers 1 nimmt die Höhe des Grates 61 zum Rand des Plattenkörpers 1 hin zu, wie am besten aus dem Vergleich der Fig. 4 mit Fig. 3 hervorgeht. Zwischen dem Grat 61 und der Rippe 33 wird eine Gangführung gebildet, die den Eingriff des Gewindes 41 der Kopfschraube 40 begünstigt.

Jedes der beiden Rundlöcher 21, 22 ist in drei Abschnitte unterteilt: Das größeren Rundloch 21 weist oberhalb der Rippe 33 einen oberen, rundkehlförmigen Abschnitt 31 mit, oder auch ohne Grat 61 auf, und unterhalb der Rippe 33 sind ein mittlerer, rundkehlförmiger Abschnitt 36 und ein unterer, kegelstumpfförmiger Abschnitt 26 vorgesehen. Das kleinere Rundloch 22 umfasst einen oberen Abschnitt 35 mit Einführungsschräge 62, einen mittleren Abschnitt 36 mit einer geneigt-abgerundeten Fläche 63 unterhalb der Ebene der Rippe 33, und einen unteren Abschnitt 28, der vorzugsweise zylindrisch ausgebildet ist, aber auch konisch sein kann.

Die Knochenplatte ist zur Zusammenwirkung mit wenigstens zweierlei Arten von Knochenschrauben ausgebildet. Die zweite Art weist einen Knochenschraubenkopf mit teilsphärischer Unterseite auf und kann sich mit der Kopfunterseite an der geneigt-abgerundeten Fläche 63 der Knochenplatte abstützen. Dabei ist auch Schrägstellung der Schraubenachse gegenüber der Plattenebene möglich, und zwar sowohl in Längsrichtung als auch (im geringen Maße) in Querrichtung zur Knochenplatte. Ermöglicht wird dies durch den Abstand der Kanten 23 voneinander, der größer ist als der Durchmesser des Schraubschaftes der Knochenschraube.

Die erste Art von anwendbarer Knochenschraube 4 ist in Fig. 4 dargestellt. Diese Knochenschraube 4 weist einen Schraubkopf 40 mit Inneneingriff und mit als Außengewinde ausgestaltetem Gewinde 41 am oberen Ende sowie konischer als Stützfläche ausgebildeter Sitzfläche 42 am unteren Ende auf, deren Konusneigung der Konusneigung der unteren Region 26 des größeren Rundloches 21 entspricht. Das Gewinde 41 kann zylindrisch sein, es wird jedoch konisches Gewinde bevorzugt.

Die Konusneigung der unteren Region 26 des größeren Rundloches 21 sowie die Konusneigung der konischen Sitzfläche 42 am unteren Ende des Schraubkopfs 40 der Knochenschraube weist einen Winkel relativ zur Längs- oder Symmetrieachse jeweils der Knochenschraube oder des Rundlochs 21 im Bereich von 3 bis 30° auf. Bevorzugt liegt dieser Konuswinkel in einem Bereich von 6 bis 20° und am bevorzugtesten in einem Bereich von 8 bis 12°. Eine besonderst bevorzugte Bauform hat sich mit einem Konuswinkel von etwa 10° mit hohen Dauerfestigkeitswerten und guter Lösbarkeit der Verbindung zwischen Knochenschraube und Knochenplatte sehr bewährt.

In einem Winkelbereich von etwa 8 bis 12° werden sehr gute Stabilitätswerte gegenüber dem Verkippen der Knochenschraube relativ zur Knochenplatte bei gleichzeitig nur moderater Selbsthemmung bereitgestellt.

An den Schraubkopf 40 schließt sich ein Schraubschaft 43 an, der dazu bestimmt ist, in einem zu fixierenden Knochenelement verankert zu werden. Die umlaufende Rippe 33 erstreckt sich in einer Ebene, vorzugsweise parallel zur Plattenebene, während sich das Gewinde 41 entlang von Schraubflächen erstreckt, die naturgemäß schräg gegenüber der Ebene der radialen Rippe 33 verlaufen, und zwar auch dann, wenn die Knochenschraube 4 bestimmungsgemäß parallel oder gleichlaufend mit der Achse 21a des Rundloches 21 verschraubt wird. Dabei erfolgt gegenseitige Formanpassung zwischen den Gewindegängen und der Rippe 33. In dieser Hinsicht ist der Grat 61 nützlich, da er ein Gegenlager für das Gewinde des Schraubkopfes bietet.

Die gegenseitige Formanpassung setzt eine gewisse Nachgiebigkeit des (ersten) Materials der Knochenplatte wenigstens im Bereich der radialen Rippe 33 und/oder des (zweiten) Materials der Knochenschraube wenigstens im Bereich des Gewindes 41 voraus. Es wird bevorzugt, das (zweite) Material der Knochenschraube härter als das (erste) Material der Knochenplatte zu machen und auch eine Form mit einem größeren Widerstandsmoment für das Gewinde 41 gegenüber dem der Rippe 33 zu wählen. Auf diese Weise passt sich die "weichere", in einer Ebene liegende Rippe weitgehend der Eingriffsschraubwindung des Gewindes 41 an, wenn das Fixationssystem bestimmungsgemäß angewendet wird. Dennoch liegt es auch im Rahmen der Erfindung, dass mindestens im Bereich der radialen Rippe 33 und des Gewindes 41 das erste und zweite Material gleiche Härten aufweisen.

Hierdurch können sowohl für die Knochenschrauben als auch für die Knochenplatte das gleiche Material verwendet werden und korrosive Effekte im Bereich und in der Nähe der Kontaktstellen von Knochenschraube mit Knochenplatte sicher vermieden werden.

Im Zusammenhang mit der dargestellten Knochenplatte kann die Knochenschraube 4 zur gegenseitigen Annährung und zum Zusammenpressen von Knochenfragmenten benutzt werden. Hierzu wird die Knochenschraube 4 mit ihrer Achse parallel zur Achse 22a im Bereich des Rundloches 22 angesetzt. Sobald der untere Rand der konischen Sitzfläche 42 die Einführungsschräge 62 des kleineren Rundloches 22 erreicht, erfolgt beim Eindrehen der Schraube eine seitliche Kraft auf den Schraubkopf 40, was zu einer Verschiebung des zu fixierenden Knochenfragments relativ zu der Knochenplatte führt. Wenn demnach ein Knochenfragment mit der Knochenplatte bereits fest verbunden ist, wird dieses Knochenfragment gegen das zu fixierende Knochenfragment geschoben, wie es erwünscht ist.

Es wird darauf hingewiesen, dass wegen des großen Umfangwinkels der Konusfläche der unteren Region 26, die im Bereich von 250 ° bis 290° liegt, bei festsitzendem Schraubkopf 40 eine genügend starke Verbindung zwischen dem Plattenkörper 1 und der Knochenschraube 4 hergestellt wird, da nach gegenseitiger Deformation die umlaufende Rippe 33 und Eingriffsschraubwindung genügend elastische Spannkraft entwickeln, um die konischen Flächen bei 26 und 42 aufeinandergepresst zu halten.

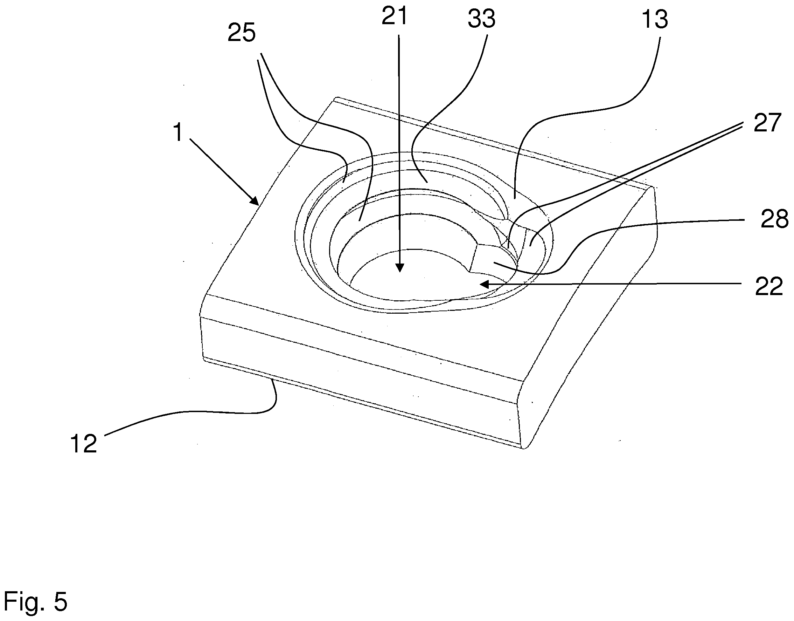

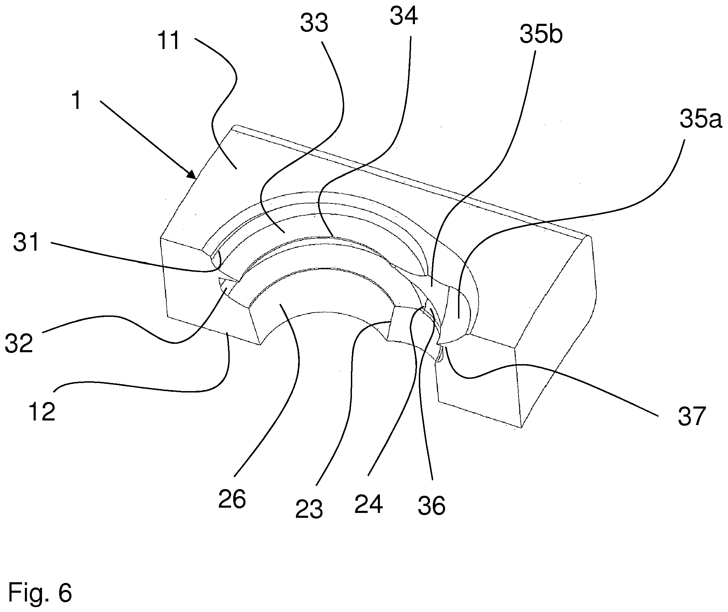

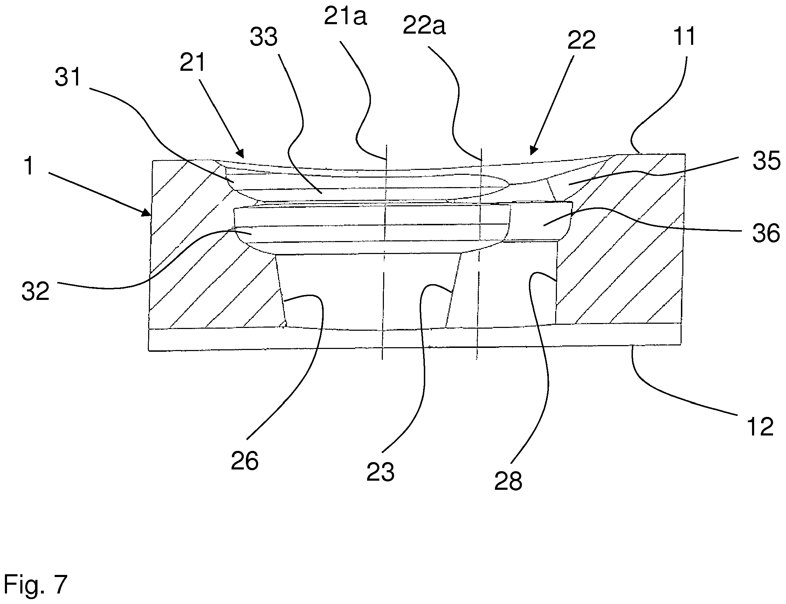

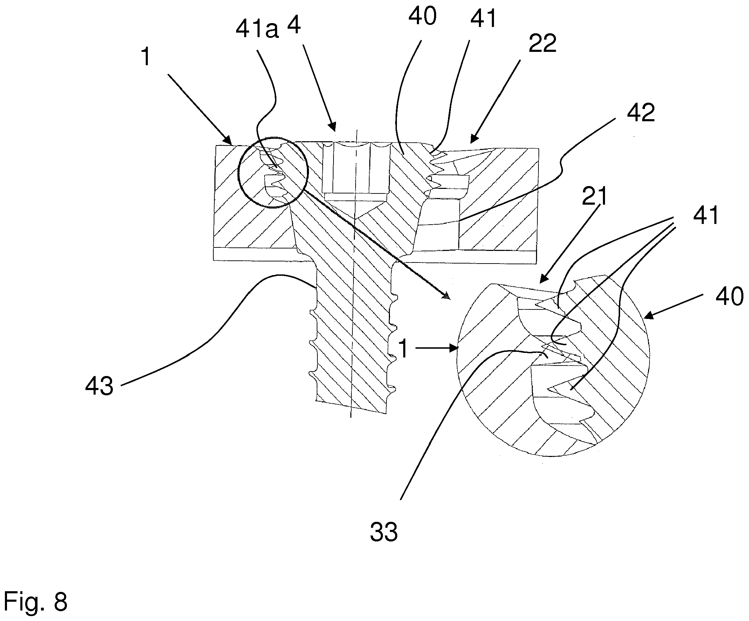

Mit Fig. 5 - 8 ist eine weitere besonders bevorzugte Ausführungsform der Knochenplatte dargestellt, wobei die gleichen Bezugszeichen für sich entsprechende Teile verwendet werden.

Der hauptsächliche Unterschied besteht in der Ausbildung der umlaufenden radialen Rippe 33. Diese ist im Bereich des kleineren Rundlochs 22 teilweise weggeschnitten, um eine geneigt-abgerundete Gleitfläche 35a und eine Übergangsfläche 35b zu schaffen, die zur Führung des Kopfes einer Knochenschraube mit teilsphärischer Unterseite des Kopfes oder zur Führung der Schraube mit konischer Sitzfläche 42 dienlich sind. Es wird eine Restrippe 37 gebildet, die auf volle Größe der Rippe 33 im Bereich des größeren Rundloches 21 ansteigt. Die verbleibende Randkante 34 erstreckt sich um weniger als 180° und erlaubt so die seitliche Einführung von Knochenschrauben vom kleineren Rundloch 22 in das größere Rundloch 21.

Die Rippe 33 hat einen keilförmigen Querschnitt mit einer zur Lochmitte geneigten Oberseite und einer Unterseite parallel zur Plattenebene. Auf diese Weise ist das Widerstandsmoment der Rippe 33 bedeutend kleiner als das Widerstandsmoment der Schraubwindung im Eingriff mit der Rippe, die Rippe 33 also nachgiebiger selbst dann, wenn die Materialien von Rippe und Schraubwindung von gleicher Härte sind. Eine geringere Härte der Rippe 33 wird jedoch zu Zwecken der Formanpassung bevorzugt.

Mit der Erfindung wird ein Fixationssystem für Knochen mit Knochenplatte und Knochenschrauben geschaffen, bei dem Knochenschrauben mit Rundkopf im unterschiedlichen Schrägwinkel zur Knochenplatte gesetzt werden können. Ferner ermöglicht das Fixationssystem bei der Anwendung von Knochenschrauben mit Kegelkopf die Relativverschiebung zwischen zu fixierendem Knochenfragment und Knochenplatte, was es dem Operateur ermöglicht, Knochenfragmente bei deren Fixierung gegeneinander zu verschieben.

## Patentansprüche

1. Fixationssystem mit Knochenplatte und Knochenschrauben, um Teile eines gebrochenen oder geschädigten Knochens zu fixieren,
wobei die Knochenplatte umfasst:
- einen vorzugsweise länglichen Plattenkörper (1), der eine Plattenebene und eine Längsachse bestimmt und aus einem ersten, insbesondere gewebeverträglichen, vorzugsweise steifen Material, besteht,
- Lochausbildungen (2) quer zur Plattenebene, die aus einem ersten Rundloch (21) und einem zweiten Rundloch (22) bestehen, welche sich unter Bildung von Kanten (23,24) schneiden und eine Schraubeneinführungsseite sowie eine Schraubenaustrittsseite besitzen, wobei das erste Rundloch (21) eine konische Sitzfläche an der Schraubenaustrittsseite aufweist, und
- eine radiale Rippe (33) in jedem der Rundlöcher, die sich in einer Ebene nahe der Schraubeneinführungsseite zur Rundlochmitte hin erstreckt,
wobei die Knochenschrauben (4) jeweils umfassen:
- einen Schraubkopf (40) aus einem zweiten, steifen Material mit im oberen Bereich des Kopfes angebrachtem Gewinde (41) und im unteren Bereich angebrachter, konischer Sitzfläche (42) sowie
- einen Schraubenschaft (43), und
wobei bei der Zusammenarbeit von Gewinde (41) und radialer Rippe (33) eine Deformation erfolgt, die eine gegenseitige Anpassung der Rippe (33) und Eingriffsgewindegang (41a) zur Folge hat.

2. Fixationssystem nach Anspruch 1,
wobei die Form und Härte der radialen Rippe (33) eine größere Nachgiebigkeit der Rippe (33) gegenüber dem Eingriffsgewindegang (41a) der Knochenschraube (4) zur Folge hat.

3. Fixationssystem nach Ansprüchen 1 bis 2,
wobei die radiale Rippe (33) von den Rundlochwandungen beider Rundlöcher (21,22) ausgeht und um diese vollständig herumführt.

4. Fixationssytem nach Anspruch 3,
wobei die umlaufende, radiale Rippe (33) im Bereich des zweiten Rundlochs (22) reduziert ist, um eine geneigt-abgerundete Gleitfläche (35a) und eine Übergangsfläche (35b) zu bilden

5. Fixationssytem nach Ansprüchen 3 oder 4,
wobei die umlaufende Rippe (33) im zweiten Rundloch (22) zur Oberseite (11) der Knochenplatte hin mit Einführungsschräge verläuft.

6. Fixationssystem nach einem der Ansprüche 1 bis 5,
wobei das erste Rundloch (21) größer als das zweite Rundloch (22) ist und umfasst:
- eine obere Region (25) in Schüsselform und mit einem zugehörigen Abschnitt der radialen Rippe (33) sowie
- eine untere Region (26) mit kegelstumpfförmiger Ausbildung und kleinerem Durchmesser gegenüber dem Durchmesser der oberen Region.

7. Fixationssystem nach Anspruch 6,
wobei das zweite, kleinere Rundloch (22) umfasst:
- eine obere Region (27) in Schüsselform und mit einem zugehörigen Abschnitt der radialen Rippe (33) sowie
- eine untere Region (28) mit einer zylindrischen oder konischen Fläche, deren Durchmesser kleiner ist als der Durchmesser der oberen Region.

8. Fixationssystem nach Anspruch 6 oder 7,
wobei die obere Region (25) des ersten, größeren Rundlochs (21) durch die Rippe (33) in einen oberen, rundkehlförmigen Abschnitt (31) oberhalb der Rippe (33) und in einen mittleren, rundkehlförmigen Abschnitt (36) unterhalb der Rippe (33) unterteilt ist und die untere Region (26) des größeren Rundlochs (21) als ein unterer, kegelstumpfförmiger Abschnitt ausgebildet ist, der sich zur Unterseite (12) des Plattenkörpers (1) hin verjüngt und dessen größter Durchmesser kleiner als der Durchmesser des oberen oder mittleren Abschnitts (31,32) ist.

9. Fixationssystem nach Anspruch 8,
wobei die obere Region (27) des zweiten, kleineren Rundloches (22) einen oberen Abschnitt (35) mit Einführungsschräge (62) aufweist und einen mittleren Abschnitt (36) mit einer geneigt-abgerundeter Fläche (63) unterhalb der Ebene der Rippe (33) besitzt.

10. Fixationssytem nach einem der Ansprüche 1 bis 9 wobei die Rippe (33) einen keilförmigen Querschnitt aufweist.

11. Knochenplatte für ein Fixationssystem nach einem der vorstehenden Ansprüche wobei die Knochenplatte umfasst:
Einen Plattenkörper (1), der eine Plattenebene und eine Längsachse bestimmt, Lochausbildungen (2) quer zur Plattenebene, die aus einem ersten Rundloch (21) und einem zweiten Rundloch (22) bestehen, welche sich unter Bildung von Kanten (23,24) schneiden und eine Schraubeneinführungsseite sowie eine Schraubenaustrittsseite besitzen, wobei das erste Rundloch (21) eine konische Sitzfläche (26) an der Schraubenaustrittsseite aufweist, und eine radiale Rippe (33) in wenigstens einem der Rundlöcher, die sich in einer Ebene nahe der Schraubeneinführungsseite zur Rundlochmitte hin erstreckt, wobei um beide Rundlöcher herum eine radiale Rippe vorgesehen ist, die von der Lochwandung ausgeht und sich in einer Ebene nahe der Schraubeneinführungsseite zur Rundlochmitte hin erstreckt.

12. Knochenplatte nach einem der vorstehenden Ansprüche,
wobei das erste Rundloch (21) größer als das zweite Rundloch (22) ist und umfasst:
eine obere Region (25) in Schüsselform und mit einem zugehörigen Abschnitt der radialen Rippe (33), sowie eine untere Region (26) mit kegelstumpfförmiger Ausbildung und kleinerem Durchmesser gegenüber dem Durchmesser der oberen Region.

13. Knochenplatte nach dem vorstehenden Anspruch,
wobei das zweite, kleinere Rundloch (22) umfasst: eine obere Region (27) in Schüsselform und mit einem zugehörigen Abschnitt der radialen Rippe (33) sowie eine untere Region (28) mit einer zylindrischen oder konischen Fläche, deren Durchmesser kleiner ist als der Durchmesser der oberen Region.

## Claims

1. A fixation system comprising a bone plate and bone screws for fixing parts of a fractured or damaged bone; wherein the bone plate comprises
- an in particular elongated plate body (1) defining a plate plane and a longitudinal axis and consisting of a first, in particular tissue-compatible, preferably rigid material,
- hole formations (2) transversely to the plate plane consisting of a first round hole (21) and a second round hole (22) which intersect to define edges (23, 24) and have a screw insertion side and a screw exit side, wherein the first round hole (21) has a conical bearing surface on the screw exit side, and
- a radial rib (33) at each of the round holes which extends in a plane near the screw insertion side towards the centre of the round hole;wherein the bone screws (4) each comprise:
- a screw head (40) made from a second, rigid material with an outer thread (41) formed in the upper region of the head and a conical bearing surface (42) formed in the lower region of the head, and
- a screw shaft (43);
wherein
the cooperation of the thread (41) and the radial rib (33) causes a deformation resulting in a mutual adaptation of the rib (33) and an engaging thread turn (41a) .

2. The fixation system according to claim 1, wherein the shape and hardness of the radial rib (33) causes a greater resilience of the rib (33) compared to the engaging thread turn (41a) of the bone screw (4).

3. The fixation system according to claims 1 to 2, wherein the radial rib (33) extends from the round hole walls of both round holes (21, 22) extending completely around the holes.

4. The fixation system according to claim 3, wherein the circumferential radial rib (33) is reduced in the region of the second round hole (22) so as to form an inclined rounded sliding surface (35a) and a transition surface (35b) .

5. The fixation system according to claims 35 or 4, wherein in the second round hole (22) the circumferential rib (33) extends with an introductory slope (62) towards the upper side (11) of the bone plate.

6. The fixation system according to any of claims 1 to 5,
wherein the first round hole (21) is greater than the second round hole (22) and comprises:
- a bowl-shaped region (25) and with an associated portion of the radial rib (33); and
- a lower region (26) in the form of a truncated cone and of smaller diameter than the diameter of the upper region.

7. The fixation system according to claim 6, wherein the second, smaller round hole (22) comprises:
- a bowl-shaped upper region (27) with an associated portion of the radial rib (33) and
- a lower region (28) with a cylindrical or conical surface having a diameter that is smaller than the diameter of the upper region.

8. The fixation system according to claim 6 or 7,
wherein the upper region (25) of the first, larger round hole (21) is divided by the rib (33) into an upper, round neck-shaped portion (31) above the rib (33) and a central, round neck-shaped portion (36) below the rib (33), and the lower region (26) of the larger round hole (21) is formed as a lower, truncated cone-shaped portion which tapers towards the lower surface (12) of the plate body (1) and has a maximum diameter which is smaller than the diameter of the upper or central portions (31, 32) .

9. The fixation system according to claim 8,
wherein the upper region (27) of the second, smaller round hole (22) comprises an upper portion (35) having an introductory slope (62) and a central portion (36) having an inclined rounded surface (63) below the plane of the rib (33).

10. The fixation system according to any of claims 1 to 9,
wherein the rib (33) has a wedge-shaped cross section.

11. Bone plate for a fixation system according to any of the precedent claims, wherein the bone plate comprises:
a body (1) defining a plane of the plate and a longitudinal axis, hole formations (2) extending transversely to the plane of the plate, the hole formations consisting of a first round hole (21) and a second round hole (22) which intersect to define edges (23, 24) and have a screw insertion side and a screw exit side, wherein the first round hole (21) has a conical bearing surface on the screw exit side, and a radial rib (33) in at least one of the round holes, which extends in a plane near the screw insertion side towards the centre of the round hole, wherein around both round holes a radial rib is provided starting from the hole wall and extending in a plane near the screw insertion side towards the centre of the round hole.

12. Bone plate according to any of the precedent claims, wherein the first round hole (21) is larger than the second round hole (22) and comprises:
an upper region (25) in bowl shape and with a section of the associated radial rib (33),
as well as a lower region (26) of frustoconical shape and smaller diameter than the diameter of the upper region.

13. Bone plate according to the precedent claim,
wherein the second, smaller round hole (22) comprises: an upper region (27) in bowl shape and with an associated section of the radial rib (33) as well as a lower region (28) with a cylindrical or conical surface, with the diameter of this surface being smaller than the diameter of the upper region.

## Revendications

1. Système de fixation comprenant une plaque osseuse et vis osseuses pour fixer des parties d'un os fracturé ou endommagé ;
dans lequel la plaque osseuse comprends:
- un corps de plaque (1), de préférence un corps de plaque (1) allongé, définissant un plan de plaque et un axe longitudinal et se composant d'un premier, en particulier compatible avec les tissus, de préférence une matériel rigide,
- configurations de trous qui sont constituées d'un premier trou rond (21) et d'un deuxième trou rond (22), lesquels sont étagés et se coupent, ce qui entraîne la formation d'arêtes (23, 24), et possèdent un côté d'introduction de vis ainsi qu'un côté de sortie de vis, et
- une nervure radiale (33) à chacun des trous ronds qui qui s'étend dans un plan dans la région supérieure (25) en direction du centre du trou;
les vis à os (4) comprenant:
- une tête (40) de vis pourvue d'un filetage (41) agencé dans la zone supérieure de la tête et d'une embase conique (42) agencée dans la zone inférieure, en ce que la tête (40) de vis se composant d'un deuxième matériel rigide , et
- d'une tige (43) de vis (43);
et dans lequel le filetage (41) et la nervure radiale (33) coopèrent, lorsque une déformation se produit, qui entraîne une adaptation mutuelle de la nervure (33) et d'un pas de filetage de contact (41a) du filetage extérieur de la vis à os (4).

2. Le système de fixation selon la revendication 1, dans lequel la forme et la dureté de la nervure radiale (33) entraînent une flexibilité accrue de la nervure (33) par rapport au pas de filetage de contact (41a) de la vis à os (4).

3. Le système de fixation selon les revendications 1 à 2, dans lequel la nervure radiale (33) fait saillie des parois des trous ronds des deux trous ronds (21, 22) et passe intégralement autour de ces derniers.

4. Le système de fixation selon la revendication3, dans lequel la nervure radiale (33) circulaire est réduite dans la zone du deuxième trou rond (22), afin de former une surface de glissement inclinée-arrondie (35a) et une surface de transition (35b).

5. Le système de fixation selon la revendication 3 ou 4, dans lequel la nervure radiale (33) circulaire est réduite dans la zone du deuxième trou rond (22), afin de former une surface de glissement inclinée-arrondie (35a) et une surface de transition (35b).

6. Le système de fixation selon l'une des revendications 1 à 5, dans lequel le premier trou rond (21) est plus grand que le deuxième trou rond (22) et sa région supérieure (25) présente une forme de cuvette et est pourvue d'une section associée de la nervure radiale (33), et la région (26) réalisée sous la forme d'une embase conique présente un diamètre inférieur au diamètre de la région supérieure.

7. Le système de fixation selon la revendication 6, dans lequel le deuxième trou rond (22) plus petit comporte une région supérieure (27) sous forme de cuvette et pourvue d'une section associée de la nervure radiale (33), ainsi qu'une région inférieure (28) présentant une surface cylindrique ou conique, dont le diamètre est inférieur au diamètre de la région supérieure.

8. Le système de fixation selon la revendication 6 ou 7, dans lequel la région supérieure (25) du premier trou (21) plus grand est divisée par la nervure (33) en une section supérieure (31) en forme de gorge ronde au-dessus de la nervure (33) et en une section centrale (36) en forme de gorge ronde au-dessous de la nervure (33) et la région inférieure (26) du trou rond (21) plus grand est réalisée sous la forme d'une section inférieure tronconique, qui se rétrécit en direction du côté inférieur (12) du corps de plaque (1) et dont le plus grand diamètre est inférieur au diamètre de la section supérieure ou centrale (31, 32).

9. Le système de fixation selon la revendication 8, dans lequel le deuxième trou rond (22) plus petit comporte une région supérieure (27) sous forme de cuvette et pourvue d'une section associée de la nervure radiale (33), ainsi qu'une région inférieure (28) présentant une surface cylindrique ou conique, dont le diamètre est inférieur au diamètre de la région supérieure.

10. Le système de fixation selon l'une des revendications 1 à 9, dans lequel la nervure (33) présente une section transversale cunéiforme.

11. Plaque osseuse pour un système de fixation selon l'une des revendications du précédent, dans lequel la plaque osseuse comprenant:
un corps de plaque (1), qui détermine un plan de plaque et un axe longitudinal,
des configurations de trous (2) transversaux au plan de plaque,
configurations de trous qui sont constituées d'un premier trou rond (21) et d'un deuxième trou rond (22), lesquels sont étagés et se coupent, ce qui entraîne la formation d'arêtes (23, 24) et possèdent un côté d'introduction de vis ainsi qu'un côté de sortie de vis, le premier trou rond (21) comprenant, sur le côté d'introduction de vis, une région supérieure (25),
au moins un trou rond comprenant une nervure radiale (33), qui partit du un paroi du trou et s'étend dans un plan dans la région supérieure (25) en direction du centre du trou.

12. Plaque osseuse selon l'une des revendications du précédent, dans lequel le premier trou rond (21) est plus grand que le deuxième trou rond (22) et sa région supérieure (25) présente une forme de cuvette et est pourvue d'une section associée de la nervure radiale (33), et la région (26) réalisée sous la forme d'une embase conique présente un diamètre inférieur au diamètre de la région supérieure.

13. Plaque osseuse selon l'une des revendications du précédent, dans lequel le deuxième trou rond (22) plus petit comporte une région supérieure (27) sous forme de cuvette et pourvue d'une section associée de la nervure radiale (33), ainsi qu'une région inférieure (28) présentant une surface cylindrique ou conique, dont le diamètre est inférieur au diamètre de la région supérieure.
